Europäisches Patentamt

European Patent Office

Office européen des brevets

(19)

(11) Publication number: **0 391 102 B1**

# EUROPEAN PATENT SPECIFICATION

(45) Date of publication of patent specification: **07.09.94**  (51) Int. Cl.⁵: **C07C 19/08**, C07C 17/00

(21) Application number: **90104927.0**

(22) Date of filing: **15.03.90**

(54) **Gas phase process for the production of 1,1-dichloro-1-fluoroethane and/or 1-chloro-1,1-difluoroethane from vinylidene chloride.**

(30) Priority: **05.04.89 US 333475**

(43) Date of publication of application:
**10.10.90 Bulletin 90/41**

(45) Publication of the grant of the patent:
**07.09.94 Bulletin 94/36**

(84) Designated Contracting States:
**AT BE CH DE DK ES FR GB GR IT LI LU NL SE**

(56) References cited:
**DE-B- 1 246 703**

(73) Proprietor: **ATOCHEM NORTH AMERICA, INC.**
**Three Parkway**
**Philadelphia, Pennsylvania 19102 (US)**

(72) Inventor: **Elsheikh, Maher Yousef**
**784 North Wayne Avenue**
**Wayne, Pennsylvania 19087 (US)**

(74) Representative: **Kraus, Walter, Dr. et al**
**Patentanwälte Kraus, Weisert & Partner**
**Thomas-Wimmer-Ring 15**
**D-80539 München (DE)**

Rank Xerox (UK) Business Services
(3.10/3.09/3.3.3)

**Description**

The present invention relates to the manufacture of 1,1-dichloro-1-fluoroethane and 1-chloro-1,1-difluoroethane by fluorination of vinylidene chloride.

1,1-Dichloro-1-fluoroethane is presently under consideration as a replacement for trichlorofluoromethane as a foam blowing agent. It has a substantially lower ozone depletion index than trichlorofluoromethane. Moreover, 1,1-dichloro-1-fluoroethane displays a 10-15% greater blowing efficiency in rigid foam, and improved solubility in aromatic polyester polyol, in comparison to trichlorofluoromethane. However, there is as yet no acceptable method for the efficient production of 1,1-dichloro-1-fluoroethane which is suitable for large scale commercial exploitation.

The liquid phase reaction of hydrogen fluoride and vinylidene chloride was reported by Henne and Plueddeman, **J. Amer. Chem. Soc.** 65, 1271 (1943). A mixture of hydrogen fluoride and vinylidene chloride was heated in a 4:1 molar ratio in an autoclave at 65° for 3 hours to give 50% 1,1-dichloro-1-fluoroethane, traces of 1-chloro-1,1-difluoroethane 10% of unreactive vinylidene chloride, 5% of 1,1,1-trichloroethane and 15% tar.

Henne and Arnold, **J. Am. Chem. Soc.** 70:758 (1945) describe the $BF_3$-catalyzed addition of hydrogen fluoride to various chlorofluoroolefins in the liquid phase at a molar ratio of HF to olefin of 2:1 at 95°C. However, the process is characterized by very low vinylidene chloride conversion, polymer formation, and lack of selectivity for the desired product.

More recently, several patents issued to Dynamit Nobel teach a gas phase, catalyzed reaction of vinylidene chloride and hydrogen fluoride. The only major product reported is 1,1,1-trifluoroethane in 98.8% to 99.8% yield, with very little or no 1,1-dichloro-1-fluoroethane produced. The reactions of vinylidene chloride and hydrogen fluoride disclosed by the aforesaid Dynamit Nobel patents are summarized in Table 1:

In 1947, McBee et al. reported the liquid phase reaction of hydrogen fluoride and vinylidene chloride in a molar ratio of from 6.6:1 to 5.4:1, in an autoclave at between 140°C and 210°C under 5,000-6,000 psi pressure, using diphenylamine as a polymerization inhibitor. McBee et al., Ind. Eng. Chem. 39, 409-12 (1947). The authors reported in Table II yields of fluorinated product up to 90%. At a temperature of 140°C the yield of fluorinated product was 76%. No 1,1-dichloro-1-fluoroethane was isolated. U.S. Patent No.

TABLE 1

| PATENT NO. | CATALYST | REACTION CONDITIONS | | PRODUCTS DISTRIBUTION (vol. %) | | | |
|---|---|---|---|---|---|---|---|
| | | TEMP.(°C) | RATIO HF:$Cl_2C=CH_2$ | $CCl_2FCH_3$ | $CClF_2CH_3$ | $CF_3CH_3$ | $Cl_2C=Cl$ |
| U.S. Patent No. 3,904,701 | $Bi(NO_3)_3$ over $Al_2O_3$ | 180 | 3.2 | 0 | 0.2 | 99.7 | 0.1 |
| | $Bi(NO_3)_3$ over $Al_2O_3$ | 205-220 | 3.7 | 0 | 0.2 | 99.8 | 0.1 |
| | $Bi(NO_3)_3$ over $Al_2O_3$ | 210-225 | 3.3 | 0 | 0.4 | 98.9 | 0.7 |
| | $Bi(NO_3)_3$ over $Al_2O_3$ | 215-225 | 3.4 | 0 | 0.2 | 99.6 | 0.2 |
| U.S. Patent No. 3,803,241 | $CrCl_3/Al_2O_3$ | 150 | 3.5 (molar) | 0.2 | 0.2 | 99.8 | 0.8 |
| British Patent No. 1,309,361 | $Mn(NO_3)_2,Bi(NO_3)_3$ over $Al_2O_3$ | 180 | 3.3 (volume) | 0 | 0.3 | 99.6 | 0.1 |
| | | 180 | 3.3 (volume) | 0 | 0.2 | 99.7 | 0.1 |

3

2,637,747 discloses a similar process.

More recently, a liquid phase process for making 1-chloro-1,1-difluoroethane was disclosed in British Patent 1,556,131. A mixture of vinylidene chloride and hydrogen fluoride was heated in the presence of catalytic amounts of SnCl₄ in an autoclave at 60°C to form 1,1-dichloro-1-fluoroethane (64.8%), 1-chloro-1,1-difluoroethane (26.7%), 1,1,1-trifluoroethane (2.1%), 1,1,1-trichloroethane (0.8%), oligomer (1.4%), and 4.2% unreacted vinylidene chloride. However, liquid phase reactions, which are generally conducted in batch rather than continuous fashion, are less desirable for economic reasons. Gas phase reactions are generally favored, as they are more readily adapted for continuous operation, and hence achieve maximum utilization of catalysts.

DE-B-1 246 703 describes the HF addition to chloroethylene, dichloroethylene, trichloroethylene and tetrachloroethylene. The process is carried out in the gas phase and the catalyst is a transition metal based catalyst composed of cobalt (2.47%), chromium (2.68%) supported on γ-alumina. Said process is characterized by a very low selectivity for the desired products.

The various prior art processes involving hydrofluorination of vinylidene chloride have relatively low selectivity for 1,1-dichloro-1-fluoroethane. Clearly what is needed is a process capable of efficient gas phase conversion of vinylidene chloride with high selectivity for 1,1-dichloro-1-fluoroethane.

The invention provides a continuous process for producing 1,1-dichloro-1-fluoroethane and 1-chloro-1,1-difluoroethane comprising continuously feeding gaseous hydrogen fluoride and gaseous vinylidene chloride to a reactor containing a solid catalyst selected from the group consisting of bismuth (III) salts, tin (IV) salts, antimony (V) salts, arsenic (III) salts, KBF₄, B₂O₃ and B(OH)₃, BF₃ on a catalyst support, and HBF₄ on a catalyst support, and continuously withdrawing from said reactor as the dominant product 1,1-dichloro-1-fluoroethane or 1-chloro-1,1-difluoroethane, whereby hydrogen fluoride and vinylidene chloride are reacted at a temperature of from 22°C to 100°C and in a molar ratio of from 1:1 to 3:1.

The process of the invention may be utilized in either batch or continuous fashion. The process is characterized by a high level conversion of vinylidene chloride to product (up to 98 mole%), with a very high selectivity for the desired product, 1,1-dichloro-1-fluoroethane. According to the process, the reaction product comprises generally at least 50 mole% of 1,1-dichloro-1-fluoroethane. Reaction selectivities for 1,1-dichloro-1-fluoroethane of 90 mole%, 98 mole%, and more, may be achieved with the present process. Moreover, few volatile by-products, substantially no tar, and substantially no oligomeric material are formed in the practice of the invention.

The process is operated between from room temperature (22°C) to 100°C.

The residence time of the reaction mixture at the reaction temperature may be selected in accordance with the desired extent of conversion of vinylidene chloride to product.

Typically, the reactants are contacted for a period of time from 10 seconds to 150 seconds, preferably from about 20 seconds to about 100 seconds.

The reaction is generally carried out at atmosphere pressure, in the presence of a suitable catalyst as defined in the claims. The catalyst comprises salts, preferably the chlorides, of the following species: bismuth (III), tin (IV), antimony (V) and arsenic (III). The chlorides are converted to the corresponding fluorides upon activation with hydrogen fluoride. Also useful are boron trifluoride, KBF₄, HBF₄, B₂O₃, B(OH)₃. The catalyst may be used directly, or may be carried on an appropriate catalyst support (boron trifluoride and HBF₄), such as activated carbon or γ-aluminum oxide. Such supported catalysts may be employed, for example, in the form of pellets or granules.

Boron trifluoride on γ-aluminum oxide is a particularly useful catalyst in the practice of the invention.

Tin(IV) salts, preferably SnCl₄, are also particularly useful. Upon hydrogen fluoride activation of activated carbon-supported SnCl₄, the resulting solid SnF₄ catalyst remains strongly adhered to the carbon support, without leaching from the catalyst bed. The catalyst remains active over the course of several days of continuous process operation.

It has been found that by selecting the appropriate reaction conditions, either 1,1-dichloro-1-fluoroethane or 1-chloro-1,1-difluoroethane may be preferentially provided. For example, using a SnCl₄ on activated carbon catalyst, selectivity for 1,1-dichloro-1-fluoroethane is obtained at lower reaction temperatures, e.g. 30°C, and at lower molar ratios of hydrogen fluoride to vinylidene chloride, e.g. 1:1. Selectivity for 1-chloro-1,1-difluoroethane is obtained at higher reaction temperatures, e.g. 70°C, and at slightly higher reactant feed ratios, e.g. 2:1. Accordingly, the process of the invention may be conveniently adapted to produce either 1,1-dichloro-1-fluoroethane or 1-chloro-1,1-difluoroethane, selectively, with high conversion (97% and greater) of vinylidene chloride.

The process may be operated with or without a carrier gas. If a carrier gas is utilized, the preferred gas for this purpose is nitrogen, in the amount of 0-20 volume percent based upon the total volume of the reactants. Other suitable carrier gases are known to those skilled in the art.

Oxygen gas may optionally be introduced into the reactor to accelerate the combustion of any carbon deposits which may accumulate on the catalyst surface as a result of burning of organic reactants. Such burning is unlikely, however, due to the relatively mild reaction conditions employed in the process.

The instant process may be carried out in a batch, semi-continuous, continuous or cyclic type process. Preferably, the process is conducted in continuous fashion, comprising continuously feeding gaseous hydrogen fluoride and gaseous vinylidene chloride into a reactor in the presence of a catalyst, and continuously withdrawing 1,1-dichloro-1-fluoroethane from the reactor. In a typical continuous operation, hydrogen fluoride and vinylidene chloride are passed through a tubular reactor loaded with hydrogen fluoride-activated catalyst. The reaction product, being predominantly 1,1-dichloro-1-fluoroethane, is removed from the reactor bottom and passed to a suitable scrubbing tower for removal of HF by the action of a countercurrent alkaline stream. The alkaline stream may comprise, for example, 1-5 N aqueous potassium hydroxide. Other aqueous hydroxides such as sodium hydroxide or calcium hydroxide may be advantageously utilized. The scrubbed product, substantially free of HF is then passed to a drying tower, packed with a suitable drying agent, e.g., anhydrous calcium sulfate.

The materials of construction of the reactor for use in the present process are not critical, except that they should possess the necessary structural and physical characteristics to withstand the reaction conditions.

The present invention is illustrated in more detail by reference to the following non-limiting examples.

## COMPARATIVE EXAMPLE 1

Harshaw pelletized aluminum oxide (50 grams) was loaded into a 2.54 cm (inside diameter) x 33.02 cm tubular reactor (Hastelloy C). The catalyst was activated for 15 hours at 650°C, using air fed to the reactor at the rate of 20 $cm^3$/minute through a valve located at the reactor top. Air activation was followed by hydrogen fluoride activation at 550°C utilizing hydrogen fluoride fed at a rate of 0.2 g/minute and nitrogen fed at a rate of 20 $cm^3$/minute. A total of 60 grams of hydrogen fluoride were fed into the reactor over 5 hours during the catalyst activation process. A mixture of hydrogen fluoride and vinylidene chloride in an 8:1 molar ratio was then fed into the reactor. The vinylidene chloride feed contained 6% oxygen and 25% nitrogen, based upon the volume of the vinylidene chloride feed. The reactor was maintained at 150°C. The reaction products were withdrawn from the bottom of the reactor after a 20 second contact time. Hydrogen fluoride was removed by passing the products through a scrubbing tower containing a continuously circulating countercurrent solution of 1-5 N aqueous potassium hydroxide. The scrubbed organic products were then passed through a drying tower packed with anhydrous calcium sulfate. The conversion of reactant to product was periodically checked by diverting product from the drying tower to a gas chromatograph equipped with an electronic integrator. The mass balance was evaluated by passing the outlet gas from the gas chromatograph through a wet test meter. Gas chromatography analysis indicated a steady 23 mole% conversion of vinylidene chloride to product, with 100% selectivity for 1,1-dichloro-1-fluoroethane.

## COMPARATIVE EXAMPLE 2

The same apparatus and procedure were used as described in Comp. Ex. 1 except that the reaction temperature was increased to 175°C. Conversion of vinylidene chloride was observed to increase to 50 mole%, with a selectivity for 1,1-dichloro-1-fluoroethane of 99.4%.

## COMPARATIVE EXAMPLE 3

The same apparatus and procedure were used as described for Comp. Ex.1 except that the contact time of the reactants in the reactor was increased to 43 seconds, the nitrogen feed was eliminated, the oxygen feed was increased to 17% based upon the volume of the vinylidene chloride feed, and the reaction temperature was increased to 175°C. Under these conditions, the conversion of vinylidene chloride was observed to increase to 65 mole%, with a selectivity for 1,1-dichloro-1-fluoroethane of 100%.

## COMPARATIVE EXAMPLE 4

Fifty grams of fluorided $\gamma$-aluminum oxide was loaded into a 2.54 cm (inside diameter) x 33.02 cm tubular reactor (Hastelloy C) and heated to 50°C. A mixture of vinylidene chloride (0.07 g) and hydrogen fluoride (0.04 g), in a 3:1 mole ratio were fed from the top of the reactor together with 0.6 $cm^3$ boron

trifluoride (1.4 molar % of the hydrogen fluoride feed). The reaction products were withdrawn from the reactor after the reactants were in contact for 25 seconds. After scrubbing hydrogen fluoride from the product stream and drying the organic product as described in Comp. Ex. 1, analysis of the product by gas chromatography as in Comp. Ex. 1 indicated 98 mole% conversion of vinylidene chloride, with 98 mole% selectivity for 1,1-dichloro-1-fluoroethane, and 2% selectivity for 1-chloro-1,1-difluoroethane.

COMPARATIVE EXAMPLE 5

Thirty grams of pelletized aluminum loaded into a 2.54 cm (inside diameter) x 33.02 cm tubular reactor (Hastelloy C) was activated with air at 650°C for 18 hours, followed by hydrogen fluoride activation at 550°C utilizing a hydrogen fluoride feed of 0.1 gram per minute for 6 hours and a $N_2$ feed of 20 $cm^3$/minute. Boron trifluoride (1.2 gram) was fed into the reactor with excess hydrogen fluoride to form $HBF_4$. A mixture of hydrogen fluoride and vinylidene chloride was then fed into the reactor as in Comp. Ex. 1 in a 3:1 molar ratio. The reaction products were withdrawn from the reactor after the reactants were in contact for 30 seconds. The product stream was scrubbed and dried as in Comp. Ex. 1. 98 mole% conversion of vinylidene chloride to product was observed, with a 1,1-dichloro-1-fluoroethane selectivity of 99 mole%.

The practice of the invention is further illustrated in the following examples, utilizing a tin(IV) chloride on activated carbon catalyst. The data from certain of these examples is listed in Table 2.

EXAMPLE 1

One hundred grams of activated carbon (Calgon CPG) was placed in a 250 ml round bottom flask interconnected to a gas inlet line and a vacuum line, and evacuated for 3 hours at 100°C. Nitrogen gas was introduced into the flask at 100°C, followed by the addition of $SnCl_4$ (101.6 g, 39 mol, 1 ml/min) at 100°C using a syringe pump, in such a way that the liquid $SnCl_4$ dripped along the wall of the flask. The $SnCl_4$ was absorbed readily by the activated carbon to form a dried $SnCl_4$ on activated carbon catalyst containing 0.0017 mole $SnCl_4$ per gram of catalyst. The catalyst (74.9 g) was loaded into the reactor described in Comp. Ex.1 and heated to 50°C with $N_2$ being added at the rate of 5 $cm^3$/min for 18 hours. Hydrogen fluoride gas and $N_2$ were fed over the catalyst at the rate of 0.1 g/min and 5 $cm^3$/min, respectively, for 2 hours. After the catalyst was completely activated, gaseous hydrogen fluoride and gaseous vinylidene chloride were fed together into the reactor in a molar ratio of 2:1 (0.03 g/min HF; 0.07 g/min vinylidene chloride) with 5 $cm^3$/min $N_2$. The reactant contact time was 82 seconds. Under these conditions, conversion of vinylidene chloride was 99.8 mole% with 68.6 mole% selectivity for 1,1-dichloro-1-fluoroethane. Additional products included 1-chloro-1,1-difluoroethane (29.5%), 1,1,1-trifluoroethane (0.9%) and 1,1,1-trichloroethane (1%). Reactor performance stayed substantially constant over the course of several days.

EXAMPLE 2

The same apparatus and procedure were used as described in Example 1, except that the molar feed ratio of hydrogen fluoride to vinylidene chloride was decreased from 2:1 to 1.1:1. Conversion of vinylidene chloride dropped slightly to 98 mole%. Selectivity for 1,1-dichloro-1-fluoroethane increased to 93 mole%. Selectivity for the other reaction products was as follows: 1-chloro-1,1-difluoroethane (5.8%), 1,1,1-trifluoroethane (0.25%) and 1,1,1-trichloroethane (0.91%).

EXAMPLE 3

The same apparatus and procedure were used as described in Example 2, except that the reactor temperature was decreased from 50°C to 30°C. The reactant contact time was 82 seconds. Conversion of vinylidene chloride remained very high (97.6 mole%), as did selectivity for 1,1-dichloro-1-fluoroethane (94.5 mole%). Selectivity for 1-chloro-1,1-difluoroethane (5.4%) and 1,1,1-trifluoroethane (0.1%) remained essentially the same. The reactor performance at 30°C at 1.1:1 molar ratio hydrogen fluoride to vinylidene chloride stayed constant for several days without any evidence of catalyst deactivation or deterioration.

EXAMPLES 4-6

A catalyst comprising 0.0027 moles of $SnCl_4$ per gram of catalyst was prepared as follows. To 100 grams of activated carbon (Calgon CPG) was slowly added 175.6 g (0.67 mol) of $SnCl_4$. The resulting

catalyst (90.8 g) was placed in the reactor described in Comp. Ex.1. The catalyst was activated by heating to 50°C, with $N_2$ being added at the rate of 5 cm$^3$/min for 18 hours, followed by hydrogen fluoride (0.1 g/min) activation at 50°C together with 5 cm$^3$/min $N_2$ for 5 hours. After the catalyst was completely activated, a mixture of hydrogen fluoride and vinylidene chloride gas in a 1:1 or 2:1 ratio at temperatures of 30, 50 and 75°C were fed into the reactor as described in Example 1 The results are shown in Table 2.

EXAMPLES 7-9

In a similar manner, a catalyst comprising 0.0027 moles SnCl$_4$ per gram of catalyst was prepared using activated carbon (Calgon BPL). The catalyst was activated as before, and its performance evaluated at 30, 50 and 75°C using a 2:1 molar feed ratio of hydrogen fluoride to vinylidene chloride. The results are summarized in Table 2.

EP 0 391 102 B1

TABLE 2

| | | | | | Conversion | PRODUCT DISTRIBUTION (mole %) | | | | |
| | | | | | CH₂=CCl₂ | | | | | |
| Example | T(°C) | Molar Ratio HF:CH₂=CCl₂ | %N₂ | Contact Time(sec.) | (mole%) | $CH_3CCl_2F$ | $CH_3CClF_2$ | $CH_3CF_3$ | $CH_3CCl_3$ | $Cl_2C=CHCl$ |
|---|---|---|---|---|---|---|---|---|---|---|
| 1 | 50 | 2:1 | 7.2 | 74 | 99.8 | 68.8 | 29.5 | 0.9 | 0 | 0 |
| 2 | 50 | 1.1:1 | 7.0 | 64 | 98.0 | 93.0 | 5.8 | 0.25 | 0.91 | 0 |
| 3 | 30 | 1.1:1 | 31.8 | 82 | 97.6 | 94.5 | 5.4 | 0.1 | 1.0 | 0 |
| - | 75 | 2.5:1 | 7.5 | 73 | 99.7 | 50.2 | 47.4 | 1.4 | 0.4 | 0.6 |
| 4 | 30 | 1:1 | 41.3 | 78 | 98.1 | 93.3 | 5.4 | 0.1 | 0.9 | 0.3 |
| 5 | 50 | 2:1 | 9.3 | 72 | 99.9 | 60.7 | 37.8 | 1.0 | 0.3 | 0.2 |
| 6 | 75 | 2:1 | 9.5 | 68 | 99.8 | 45.1 | 52.3 | 1.9 | 0.4 | 0.3 |
| 7 | 30 | 2:1 | 8.1 | 58 | 99.8 | 83.4 | 15.5 | 0.4 | 0.6 | 0.2 |
| 8 | 50 | 2:1 | 7.4 | 66 | 98.7 | 73.4 | 24.6 | 1.3 | 0.4 | 0.3 |
| 9 | 75 | 2:1 | 7.6 | 63 | 98.2 | 13.4 | 74.8 | 10.8 | 0.2 | 0.8 |

Particularly high selectivity for 1,1-dichloro-1-fluoroethane was achieved with a tin (IV) chloride catalyst at 30°C. At this temperature, selectivities for 1,1-dichloro-1-fluoroethane of 93.1, 93.3 and 83.4 mole%, from 1.1:1, 1:1 and 2:1 hydrogen fluoride to vinylidene chloride molar feed ratios, respectively. Moreover, the reaction is characterized by an extremely high vinylidene chloride conversion, being 97.6 mole% and greater.

8

Examples 10-13 illustrate the practice of the invention utilizing a 0.0017 mole/g SnCl$_4$ catalyst in the absence of a carrier gas.

EXAMPLE 10

Activated carbon (100 grams, Calgon CPG) placed in a 250 ml round bottomed flask, which was interconnected into a nitrogen gas inlet and vacuum line, was evacuated for three hours at 100°C. After cooling the flask to room temperature, nitrogen gas was admitted to the flask followed by the addition of 200 ml of methylene chloride. SnCl$_4$ (101.6 grams, 0.39 mole) was added to the stirred suspension of the activated carbon in methylene chloride solution at a feed rate of 1 ml/min. After complete addition (45 minutes), the reaction mixture was further stirred at room temperature for one hour. The methylene chloride solvent was driven off by distillation at atmospheric pressure, and the reaction mixture was further dried using a vacuum pump at 50°C. From the catalyst obtained, 40 grams were loaded into the reactor described in Comp. Ex. 1 with 5 cm$^3$/min N$_2$ for 18 hours, followed by hydrogen fluoride (0.1 g/min) and nitrogen (5 cm$^3$/min) for two hours. Feeding a mixture of HF (0.03 g/min) and vinylidene chloride (0.07 g/min) at 75°C, gave 99.7% conversion of vinylidene chloride. The product distribution, in mole percent, was as follows:

| 1,1-dichloro-1-fluoroethane | 25.8 % |
|---|---|
| 1-chloro-1,1-difluoroethane | 70.2 % |
| 1,1,1-trifluoroethane | 2.98 % |
| 1,1,1-trichloroethane | 0.19 % |
| trichloroethylene | 0.88 % |

EXAMPLE 11

The same catalyst and reactor feed of Example 10 were employed, except that the reaction temperature was lowered to 50°C. A 99.74 mole% conversion of vinylidene chloride was observed. Selectivity for 1,1-dichloro-1-fluoroethane increased to 88.1 mole%. The product distribution in mole percent, was as follows:

| 1,1-dichloro-1-fluoroethane | 88.1 % |
|---|---|
| 1-chloro-1,1-difluoroethane | 10.23 % |
| 1,1,1-trifluoroethane | 0.31 % |
| 1,1,1-trichloroethane | 0.76 % |
| trichloroethylene | 0.6 % |

EXAMPLE 12

The same catalyst from Example 10 was employed. The reaction was run at 30°C with a reactant feed of 0.07 g/min vinylidene chloride and 0.02 g/min HF. Conversion of vinylidene chloride remained very high, 99.85 mole%. The product distribution in mole percent, was as follows:

| 1,1-dichloro-1-fluoroethane | 68.4 % |
|---|---|
| 1-chloro-1,1-difluoroethane | 30.2 % |
| 1,1,1-trifluoroethane | 0.44 % |
| 1,1,1-trichloroethane | 0.21 % |
| trichloroethylene | 0.3 % |

The processes described in each of Examples 11, 12 and 13 was run continuously for eleven days without evidence of catalyst deactivation.

Examples 13-14 illustrate the practice of the invention utilizing a catalyst comprising 0.0017 mole% SbCl$_5$ per gram of catalyst.

EXAMPLE 13

Activated carbon (105 grams, Calgon CPG) placed in a 250 ml round bottomed flask, which was interconnected into a nitrogen gas inlet and a vacuum line, was evacuated for four hours at 100°C. After completing the evacuation, the vacuum line was disconnected and nitrogen gas was admitted to the flask at 100°C. Antimony (V) chloride (117 g, 0.39 mole) was gradually added to the heated activated carbon with continuous shaking of the flask in such a way that $SbCl_5$ was dripping on the wall of the flask. The dried catalyst (69.3 grams) was activated with nitrogen at 50°C for 18 hours, fed at a rate of 5 cm$^3$/min, followed by HF activation with 1 g/min HF for four hours. The catalyst performance was evaluated at 50°C in the hydrofluorination of vinylidene chloride in the same reactor used in Comp. Ex. 1 at a feed rate of 0.03 g/min HF, 0.07 g/m vinylidene chloride and 10 cm$^3$/min of $N_2$. The reaction was run continuously for two days. Fifty mole% conversion of vinylidene chloride was observed, with a selectivity for 1,1-dichloro-1-fluoroethane of 93.4 mole%. The product distribution in mole percent, was as follows:

| 1,1-dichloro-1-fluoroethane | 93.4 % |
|---|---|
| 1-chloro-1,1-difluoroethane | 3.3 % |
| 1,1,1-trifluoroethane | 0.1 % |
| 1,1,1-trichloroethane | 2.7 % |
| trichloroethylene | 0.5 % |

EXAMPLE 14

The reaction of Example 13 was repeated except that the temperature was raised from 50°C to 75°C. Conversion of vinylidene chloride increased to 63 mole%. The product distribution, in mole percent, was as follows:

| 1,1-dichloro-1-fluoroethane | 94.5 % |
|---|---|
| 1-chloro-1,1-difluoroethane | 3.9 % |
| 1,1,1-trifluoroethane | 0.8 % |
| trichloroethylene | 0.8 % |

**Claims**

1. A continuous process for producing 1,1-dichloro-1-fluoroethane and 1-chloro-1,1-difluoroethane comprising continuously feeding gaseous hydrogen fluoride and gaseous vinylidene chloride to a reactor containing a solid catalyst selected from the group consisting of bismuth (III) salts, tin (IV) salts, antimony (V) salts, arsenic (III) salts, $KBF_4$, $B_2O_3$ and $B(OH)_3$, $BF_3$ on a catalyst support, and $HBF_4$ on a catalyst support, and continuously withdrawing from said reactor as the dominant product 1,1-dichloro-1-fluoroethane or 1-chloro-1,1-difluoroethane, whereby hydrogen fluoride and vinylidene chloride are reacted at a temperature of from 22°C to 100°C and in a molar ratio of from 1:1 to 3:1.

2. The process according to claim 1 wherein the catalyst is $SnCl_4$.

3. The process according to claim 2 wherein the catalyst is supported on a catalyst support comprising activated carbon.

4. The process according to claims 1 to 3 wherein the hydrogen fluoride and vinylidene chloride are in contact for a period of time from 10 seconds to 150 seconds.

5. The process according to claims 1 to 4 wherein the hydrogen fluoride and vinylidene chloride are in contact for a period of time from 20 seconds to 100 seconds.

10

**Patentansprüche**

1. Kontinuierliches Verfahren zur Herstellung von 1,1-Dichlor-1-fluorethan und 1-Chlor-1,1-difluorethan, dadurch **gekennzeichnet,** daß man kontinuierlich gasförmigen Fluorwasserstoff und gasförmiges Vinylidenchlorid in einen Reaktor einleitet, der einen festen Katalysator, ausgewählt aus der Gruppe, bestehend aus Wismut(III)-Salzen, Zinn(IV)-Salzen, Antimon(V)-Salzen, Arsen(III)-Salzen, $KBF_4$, $B_2O_3$ und $B(OH)_3$, $BF_3$ auf einem Katalysatorträger und $HBF_4$ auf einem Katalysatorträger, enthält, und daß man kontinuierlich aus dem Reaktor als Hauptprodukt 1,1-Dichlor-1-fluorethan oder 1-Chlor-1,1-difluorethan abnimmt, wobei Fluorwasserstoff und Vinylidenchlorid bei einer Temperatur von 22°C bis 100°C und in einem Molverhältnis von 1:1 bis 3:1 miteinander umgesetzt werden.

2. Verfahren nach Anspruch 1, dadurch **gekennzeichnet,** daß der Katalysator $SnCl_4$ ist.

3. Verfahren nach Anspruch 2, dadurch **gekennzeichnet,** daß der Katalysator von einem Katalysatorträger getragen wird, der aktivierten Kohlestoff umfaßt.

4. Verfahren nach den Ansprüchen 1 bis 3, dadurch **gekennzeichnet,** daß sich der Fluorwasserstoff und das Vinylidenchlorid über einen Zeitraum von 10 Sekunden bis 150 Sekunden in Kontakt befinden.

5. Verfahren nach den Ansprüchen 1 bis 4, dadurch **gekennzeichnet,** daß sich der Fluorwasserstoff und das Vinylidenchlorid über einen Zeitraum von 20 Sekunden bis 100 Sekunden in Kontakt befinden.

**Revendications**

1. Procédé continu de production de 1,1-dichloro-1-fluoréthane et de 1-chloro-1,1-difluoréthane, comprenant l'introduction continue de fluorure d'hydrogène gazeux et de chlorure de vinylidène gazeux dans un réacteur contenant un catalyseur solide choisi dans le groupe consistant en sels de bismuth (III), sels d'étain (IV), sels d'antimoine (V), sels d'arsenic (III), $KBF_4$, $B_2O_3$ et $B(OH)_3$, $BF_3$ sur un support de catalyseur et $HBF_4$ sur un support de catalyseur, et la décharge continue de ce réacteur, comme produit dominant, de 1,1-dichloro-1-fluoréthane ou de 1-chloro-1,1-difluoréthane, le fluorure d'hydrogène et le chlorure de vinylidène étant amenés à réagir à une température de 22°C à 100°C et dans un rapport molaire de 1:1 à 3:1.

2. Procédé suivant la revendication 1, dans lequel le catalyseur est $SnCl_4$.

3. Procédé suivant la revendication 2, dans lequel le catalyseur est fixé sur un support comprenant du carbone activé.

4. Procédé suivant les revendications 1 à 3, dans lequel le fluorure d'hydrogène et le chlorure de vinylidène restent en contact pendant une période allant de 10 secondes à 150 secondes.

5. Procédé suivant les revendications 1 à 4, dans lequel le fluorure d'hydrogène et le chlorure de vinylidène restent en contact pendant une période allant de 20 secondes à 100 secondes.